(19) Europäisches Patentamt
European Patent Office
Office européen des brevets

(11) Veröffentlichungsnummer : **0 171 046**
**B1**

(12) # EUROPÄISCHE PATENTSCHRIFT

(45) Veröffentlichungstag der Patentschrift : **09.11.88**

(51) Int. Cl.[4] : **C 07 D307/32**

(21) Anmeldenummer : **85109746.9**

(22) Anmeldetag : **02.08.85**

(54) **Verfahren zur Herstellung von Pantolacton.**

(30) Priorität : **10.08.84 DE 3429439**

(43) Veröffentlichungstag der Anmeldung : **12.02.86 Patentblatt 86/07**

(45) Bekanntmachung des Hinweises auf die Patenterteilung : **09.11.88 Patentblatt 88/45**

(84) Benannte Vertragsstaaten : **CH DE FR GB IT LI**

(56) Entgegenhaltungen :
**CHEMICAL ABSTRACTS, Band 93, Nr. 1, 7. Juli 1980, Seite 832, Zusammenfassung Nr. 239197b, Columbus, Ohio, US**

(73) Patentinhaber : **BASF Aktiengesellschaft**
**Carl-Bosch-Strasse 38**
**D-6700 Ludwigshafen (DE)**

(72) Erfinder : **Mesch, Walter, Dr.**
**Richinesstrasse 11**
**D-6700 Ludwigshagen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 171 046 B1

Jouve, 18, rue St-Denis, 75001 Paris, France

**Beschreibung**

Die vorliegende Erfindung betrifft ein Verfahren zur Herstellung von Pantolacton aus einem Salz der Glyoxylsäure und Isobutyraldehyd, Reduktion des Umsetzungsproduktes und Lactonisierung.

Aus der JA-OS 80/62080 ist bekannt, daß sich Pantolacton (das Lacton der 2,4-Dihydroxy-3,3-dimethyl-buttersäure) herstellen läßt, indem man das Natrium- oder Kaliumsalz der Glyoxylsäure mit Isobutyraldehyd umsetzt und das Aldolisierungsprodukt in Gegenwart von im Überschuß eingesetzter Glyoxylsäure und Lauge zum Alkalisalz der 2,4-Dihydroxy-3,3-dimethylbuttersäure reduziert. Das hierbei entstehende Natrium- bzw. Kaliumoxalat muß durch technisch aufwendige Filtration entfernt werden. Wird in Gegenwart von Formaldehyd statt Glyoxylsäure reduziert, so gehen die Ausbeuten deutlich zurück.

Zur nachfolgenden Lactonisierung ist es notwendig, die Reaktionslösung anzusäuern, was zur Freisetzung von erheblichen Mengen an Salzen führt.

Der Erfindung lag daher die Aufgabe zugrunde, das beschriebene Verfahren einfacher und wirtschaftlicher zu gestalten und die Salzbildung, die hinsichtlich der damit verbundenen Umweltbelastung unbefriedigend ist, zu umgehen.

Demgemäß wurde ein Verfahren zur Herstellung von Pantolacton aus einem Salz der Glyoxylsäure und Isobutyraldehyd, anschließende Reduktion des Umsetzungsproduktes und Lactonisierung gefunden, welches dadurch gekennzeichnet ist, daß man

a) ein Salz der Glyoxylsäure mit einer tertiären Stickstoffbase mit Isobutyraldehyd umsetzt,
b) das so erhaltene Umsetzungsprodukt katalytisch hydriert und
c) das Hydrierprodukt erwärmt.

Das Gelingen des Verfahrens, das nach folgendem Reaktionsschema verläuft :

a)

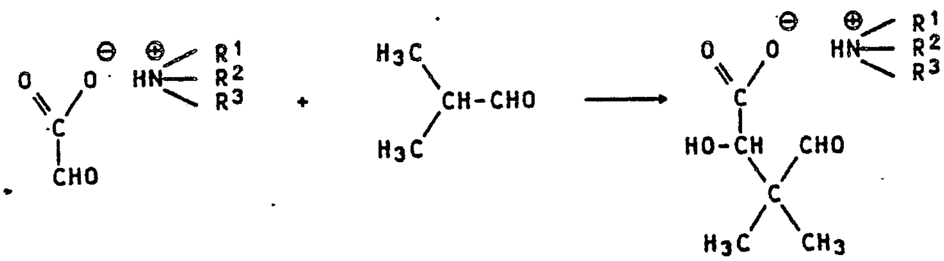

(I) (II) (III)

b) (III)

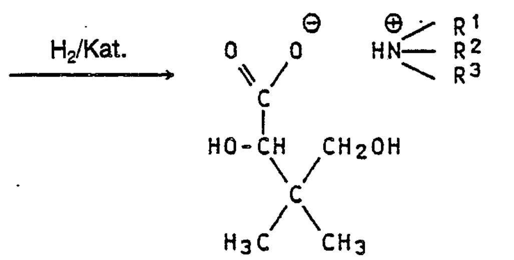


(IV)

Erwärmen

c)

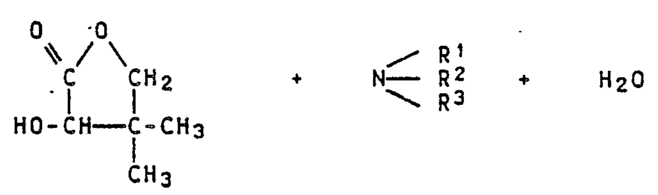

(V)

ist bemerkenswert, da nicht vorherzusehen war, daß Verbindung (IV) beim Erwämen spontan unter Abspaltung der tertiären Stickstoffbase cyclisieren würde, so daß sich das Ansäuern der Reaktionslösung erübrigt und die damit verbundene Salzbildung fortfällt. Der mit der unerwarteten Abspaltung des

tertiären Amins verbundene weitere Vorteil liegt darin, daß bei Wahl einer flüchtigen Aminkomponente diese wieder leicht dem Schritt der Ammoniumsalzbildung zurückgeführt werden kann.

Für die Umsetzung mit Glyoxylsäure lassen sich prinzipiell solche tertiäre Stickstoffbasen einsetzen, die cycloaliphatische Reste, beispielsweise Cycloalkylreste mit bis zu 6 Ringgliedern wie Cyclohexyl- oder Cyclopentylgruppen, Aralkylreste mit 7 bis 12 Kohlenstoffatomen oder Phenylreste enthalten. Heteroaromaten wie z. B. Pyridin sind aufgrund ihrer geringeren Basizität weniger gut geeignet als substituierte tertiäre Amine der allgemeinen Formel

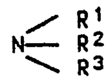

Hier kommen insbesondere Amine in Betracht, in denen $R^1$, $R^2$ und $R^3$ für aliphatische Reste mit jeweils 1 bis 12 Kohlenstoffatomen, insbesondere für Alkylreste mit 1 bis 12, vorteilhaft mit 1 bis 8 Kohlenstoffatomen, stehen. Die Reste können sowohl verzweigt als auch unverzweigt oder untereinander zu einem Ring geschlossen sein wie z. B. im N-Methylpyrrolidin, der zudem noch ein weiteres Heteroatom tragen kann wie z. B. im N-Methylmorpholin.

Besonders geeignet sind $C_1$-$C_4$-alkylsubstituierte tertiäre Amine wie z. B. Tripropyl-, Triethyl- oder Trimethylamin.

Von diesen zeichnet sich das Trimethylamin dadurch aus, daß es bei Normaltemperatur gasförmig ist. Es erweist sich deshalb als besonders vorteilhaft, weil es mit einem geringen Aufwand an Energie wieder in die Reaktionsstufe der Salzbildung zurückgeführt werden kann.

Die Menge des vorzugsweise bei einer Temperatur von 10 bis 80 °C zugesetzten tertiären Amins liegt in der Regel bei 1 bis 1,2 mol pro Mol Glyoxylsäure. Sie kann darüber hinaus erhöht oder herabgesetzt werden. Weniger als äquimolare Mengen führen zu erhöhten Reaktionszeiten bei der Umsetzung von (I) mit (II).

Die nachfolgende Aldolisierung zu (III) erfolgt vorzugsweise bei 20 bis 100, insbesondere bei 40 bis 80 °C.

Die katalytische Hydrierung von (III) wird vorzugsweise bei Temperaturen von 50 bis 150, in der Regel 80 bis 100 °C in Gegenwart eines geeigneten Hydrierkatalysators nach den dafür üblichen Techniken durchgeführt. Bewährt hat sich ein Kupferkatalysator auf Trägern wie Aluminiumoxid und Hydrierung unter Druck bei vorzugsweise 250 bar Wasserstoff in einem kontinuierlich betriebenen Röhrenreaktor.

Nach der Hydrierung wird das tertiäre Amin, sofern nicht schon abgespalten, durch Erwärmen des Reaktionsgemisches bis auf maximal 200 °C je nach Aminkomponente freigesetzt.

Die Rückgewinnung der Aminkomponente und die Isolierung von (V) geschieht in der Regel durch destillative Aufarbeitung des nach der Hydrierung erhaltenen rohen Reaktionsgemisches nach den dafür üblichen Techniken.

Das beschriebene Verfahren nach der Erfindung liefert im Vergleich zum Stand der Technik das Wertprodukt (V) auf einfacherem und wirtschaftlicherem Weg in hoher isolierter Ausbeute und hohem Reinheitsgehalt. Es ist vergleichsweise gerade für den Betrieb im großtechnischen und kontinuierlichen Maßstab geeignet und ist umweltfreundlich.

Das Pantolacton (V) ist ein wertvoller Ausgangsstoff für die Herstellung von Pharmazeutika und Vitaminen, insbesondere von Pantothensäure. Bezüglich der Verwendung wird auf Ullmanns Encyclopädie der technischen Chemie, Band 18, Seiten 201 bis 203 verwiesen.

## Beispiel 1

5 920 g (40 mol) wäßrige, 50 %ige Glyoxylsäure wurden bei einer Temperatur von 30 bis 40 °C mit 4 450 g (44 mol) Triethylamin versetzt. In die so erhaltene Lösung des Triethylammoniumglyoxylats wurden bei 40 °C 2 880 g (40 mol) Isobutyraldehyd gegeben und anschließend die Reaktionstemperatur des zweiphasigen Gemisches auf 60 °C erhöht. Nach 2 Stunden wurde die dann homogene Lösung in einem Röhrenreaktor bei 80 °C und 250 bar Wasserstoff hydriert, indem man sie kontinuierlich über einen Kupferkontakt in Rieselfahrweise leitete.

Anschließend wurde das Hydrierungsprodukt destilliert. Dabei ging im Normaldruck bei Temperaturen bis 100 °C ein Gemisch von Wasser und Triethylamin über. 95 % der eingesetzten Triethylaminmenge wurden so zurückerhalten.

Durch Fortsetzung der Destillation bei vermindertem Druck wurden aus 1 000 g ursprünglichem Hydrieraustrag 353 g (27 mol) Pantolacton, entsprechend 90 % der Theorie, mit einer gaschromatographisch ermittelten Reinheit von 98 % erhalten.

## Beispiel 2

In 2 960 g (20 mol) wäßrige, 50 %ige Glyoxylsäure wurde bei 40 °C Trimethylamin bis zur Sättigung eingeleitet, wobei der pH-Wert der Lösung von anfangs 1,0 auf schließlich 9,5 stieg.

Das so erhaltene Trimethylammoniumglyoxylat wurde mit 1 440 g (20 mol) Isobutyraldehyd versetzt und 2 Stunden bei 40 °C, sowie 12 Stunden bei 60 °C gerührt.

Nach der analog Beispiel 1 durchgeführten Hydrierung wurde das Trimethylamin durch Erwärmen des Reaktionsgemisches auf 120 °C abgespalten und in einer Kühlfalle kondensiert, dabei wurde über 90 % der ursprünglich eingesetzten Menge zurückerhalten.

Die Destillation lieferte 2 420 g (18,6 mol) Pantolacton, entsprechend 93 % der Theorie.

**Patentansprüche**

1. Verfahren zur Herstellung von Pantolacton aus einem Salz der Glyoxylsäure und Isobutyraldehyd, anschließende Reduktion des Umsetzungsproduktes und Lactonisierung, dadurch gekennzeichnet, daß man
   a) ein Salz der Glyoxylsäure mit einer tertiären Stickstoffbase mit Isobutyraldehyd umsetzt.
   b) das so erhaltene Umsetzungsprodukt katalytisch hydriert und
   c) das Hydrierprodukt erwärmt.
2. Verfahren nach Anspruch 1, dadurch gekennzeichnet, daß man die während b) und/oder c) abgespaltene tertiäre Stickstoffbase in die Stufe a) wieder zurückgeführt.
3. Verfahren nach Anspruch 1, dadurch gekennzeichnet, daß man als Salz der Glyoxylsäure ein Salz mit einem Tri-($C_1$-$C_4$) alkylamin verwendet.
4. Verfahren nach Anspruch 1, dadurch gekennzeichnet, daß man das Trimethylammoniumsalz der Glyoxylsäure umsetzt.

**Claims**

1. A process for the preparation of pantolactone from a salt of glyoxylic acid and isobutyraldehyde, with subsequent reduction of the reaction product and lactonization, wherein
   a) a salt of glyoxylic acid with a tertiary nitrogen base is reacted with isobutyraldehyde,
   b) the reaction product thus obtained is hydrogenated catalytically and
   c) the hydrogenation product is heated.
2. A process as claimed in claim 1, wherein the tertiary nitrogen base eliminated in the course of b) and/or c) is recycled to stage a).
3. A process as claimed in claim 1, wherein the glyoxylic acid salt used is a salt with a tri-($C_1$-$C_4$)-alkylamine.
4. A process as claimed in claim 1, wherein the trimethylammonium salt of glyoxylic acid is reacted.

**Revendications**

1. Procédé de préparation de pantolactone à partir d'un sel d'acide glyoxylique et d'isobutyraldéhyde, réduction ultérieure du produit de réaction et lactonisation, caractérisé en ce que
   a) on fait réagir avec l'isobutyraldéhyde un sel d'acide glyoxylique et d'une base azotée tertiaire,
   b) on hydrogène catalytiquement le produit de réaction ainsi obtenu, et
   c) on chauffe le produit d'hydrogénation.
2. Procédé selon la revendication 1, caractérisé en ce qu'on recycle à l'étape a) la base azotée tertiaire séparée au cours de b) et/ou c).
3. Procédé selon la revendication 1, caractérisé en ce qu'on utilise comme sel de l'acide glyoxylique un sel formé avec une tri-(alkyl en $C_1$-$C_4$) amine.
4. Procédé selon la revendication 1, caractérisé en ce qu'on fait réagir le glyoxylate de triméthylammonium.